# EUROPÄISCHE PATENTSCHRIFT

(11) **EP 3 137 475 B1**
(45) Veröffentlichungstag und Bekanntmachung des Hinweises auf die Patenterteilung: **27.06.2018**
(21) Anmeldenummer: 15725239.6
(22) Anmeldetag: 30.04.2015
(51) Int. Cl.: C07H 15/24

(54) **AUFREINIGUNG VON EPIDAUNORUBICIN**
PURIFICATION OF EPIDAUNORUBICIN
PURIFICATION D'ÉPIDAUNORUBICINE

(30) Priorität: 30.04.2014 DE 102014208194
(43) Veröffentlichungstag der Anmeldung: 08.03.2017
(73) Patentinhaber: medac Gesellschaft für klinische Spezialpräparate mbH, 22880 Wedel (DE)
(72) Erfinder: BINDERNAGEL, Holger, 63571 Gelnhausen (DE); KUNNARI, Tero, 63739 Aschaffenburg (DE)
(74) Vertreter: Vossius & Partner Patentanwälte Rechtsanwälte mbB
(86) Internationale Anmeldenummer: PCT/EP2015/059441
(87) Internationale Veröffentlichungsnummer: WO 2015/166016

(56) Entgegenhaltungen:
- EP-A1- 1 990 405
- EP-B1- 2 042 608
- WO-A1-2010/028667

## Beschreibung

Die vorliegende Erfindung betrifft ein Verfahren zur Aufreinigung von Epidaunorubicin, insbesondere die Trennung von Epidaunorubicin und epi-Feudomycin, wie es bei der biotechnologischen Herstellung von Epidaunorubicin als Nebenprodukt auftritt.

Epidaunorubicin ist ein 4'-Epimer des Daunorubicins, das zur Gruppe der Glycoside gehört und ein Antibiotikum aus der Gruppe der Anthracycline darstellt. Es dient hauptsächlich als Vorstufe für Epirubicin, das als Zytostatikum bei der Chemotherapie von Brustkrebs, Non-Hodgkin-Lymphomen, Sarkomen, Magenkarzinomen und anderen soliden Krebsarten eingesetzt wird. Epidaunorubicin kann synthetisch, semisynthetisch und biotechnologisch hergestellt werden. Biotechnologisch wird es mit Hilfe verschiedener Streptomyces peucetius-Stämme hergestellt. Epidaunorubicin kann durch die folgende allgemeine Formel (I) dargestellt werden:

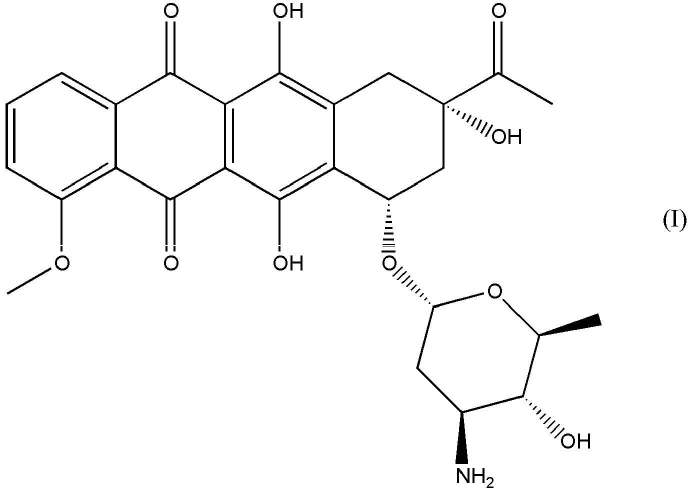

Bei der mikrobiellen Synthese des Epidaunorubicins ergibt sich das Problem, dass neben dem gewünschten Produkt unter anderem epi-Feudomycin als Nebenprodukt gebildet wird, das sich aufgrund der strukturellen Ähnlichkeit nur schwer vom Epidaunorubicin abtrennen lässt. Die Anwesenheit des Nebenproduktes wirkt sich nachteilig auf die Ausbeute und den Reinheitsgrad des in einem weiteren Verfahrensschritt aus dem Epidaunorubicin gebildeten Epirubicins aus. Üblicherweise erfolgt die Abtrennung und Reinigung des Epidaunorubicin aus der Fermentationsbrühe mittels flüssig-flüssig-Extraktion, Chromatographie und Kristallisation.

Dies ist allerdings aufgrund der Anwesenheit des epi-Feudomycins mit einem hohen Aufwand und einem verhältnismäßig großen Verlust an Epidaunorubicin verbunden.

EP 1 990 405 A1 beschreibt verschiedene mikrobielle Stämme, die für die biotechnische Herstellung von Epidaunorubicin geeignet sind. Die Abtrennung des Epidaunorubicins aus der Fermentationsbrühe erfolgt durch Extraktion mit Chloroform bei einem alkalischen pH-Wert. Die erhaltene Rohmischung wird anschließend mit Chloroform als mobile Phase chromatographisch weiter aufgereinigt. In einem letzten Schritt wird das Epidaunorubicin durch Zugabe von Butanol und Einstellen eines sauren pH-Wertes auskristallisiert.

EP 2 301 943 B1 beschreibt die Kristallisation vom Epidaunorubicin in Form seines Hydrochloridsalzes aus einem Alkohol/Chloroform-Gemisch, wobei die Zugabe des Alkohols bei 60 °C erfolgt.

EP 0 030 295 B1 offenbart die synthetische Herstellung von Epidaunorubicin.

WO 2010/028667 beschreibt die Gewinnung von 13-DHED, Epidaunorubicin und epi-Feudomycin aus einer Fermentationsbrühe mit Hilfe eines Austauscherharzes.

EP 2 042 608 B1 beschreibt die Extraktion von Aglyconen aus einer Fermentationsbrühe enthaltend 13-DHED, Epidaunorubicin und Feudomycin. Dabei werden die Glycoside mittels Chloroform aus der wässrigen Phase bei einem leicht-basischen pH-Wert extrahiert. Der pH-Wert wird dabei mit Hilfe von gesättigter NaHCO₃-Lösung stabil gehalten.

Die üblichen Verfahren zur Aufreinigung von Epidaunorubicin aus der Fermenationsbrühe sind mit einem hohen technischen und finanziellen Aufwand verbunden. Insbesondere die Separation von Epidaunorubicin und epi-Feudomycin gestaltet sich aufgrund der strukturellen Ähnlichkeit der beiden Verbindungen als sehr schwierig, so dass ein annehmbarer Reinheitsgrad des Epidaunorubicin nur in Verbindung mit einem erheblichen Ausbeuteverlust erreicht werden kann. Bedingt durch die schwierige Abtrennung des epi-Feudomycins findet sich diese Verunreinigung auch in den Folgeprodukten des Epidaunorubicins, was sich insbesondere störend auf die Umsetzung zu Epirubicin auswirkt. Andererseits sind gerade ein hoher Reinheitsgrad und eine hohe Ausbeute entscheidend für die weitere Umsetzung von Epidaunorubicin zu Epirubucin.

Es besteht daher Bedarf an einem Verfahren, das eine effektive Trennung des Nebenproduktes epi-Feudomycin von dem gewünschten Produkt Epidaunorubicin erlaubt, ohne dass die Ausbeute an Epidaunorubicin durch die Trenn- und Reinigungsschritte signifikant erniedrigt wird.

Es ist daher Aufgabe der vorliegenden Erfindung, ein Verfahren bereitzustellen, das eine effektive Trennung von Epidaunorubicin und epi-Feudomycin nach der mikrobiellen Herstellung erlaubt, wobei die Ausbeute an gereinigtem Epidaunorubicin gegenüber den im Stand der Technik üblichen Verfahren gesteigert ist.

Die Aufgabe wird durch den Gegenstand des unabhängigen Anspruchs gelöst. Bevorzugte Ausführungsformen sind in den abhängigen Ansprüchen beschrieben.

Ein Gegenstand der vorliegenden Erfindung ist ein Verfahren zur Aufreinigung von Epidaunorubicin umfassend die folgenden Schritte:
a) Bereitstellen einer Mischung enthaltend Epidaunorubicin, epi-Feudomycin und wenigstens ein halogenhaltiges Lösungsmittel;
b) Einstellen des pH-Wertes der Mischung auf einen Bereich von 5,0 bis 7,5;
c) Erwärmen der Mischung aus Schritt b) auf über 50 °C; und
d) Aufreinigen des Epidaunorubicins,
dadurch gekennzeichnet, dass der Anteil an Alkoholen mit 1 bis 5 Kohlenstoffatomen an der Mischung in den Schritten a) und b) maximal 5 Vol.-%, bezogen auf das Gesamtvolumen der Mischung, beträgt.

Ohne an eine Theorie gebunden zu sein, wird vermutet, dass es unter den Bedingungen des erfindungsgemäßen Verfahrens zu einer selektiven Zersetzung des epi-Feudomycins kommt, dessen Zersetzungsprodukte sich jedoch einfacher von dem gewünschten Epidaunorubicin abtrennen lassen. Es wird vermutet, dass es beim erfindungsgemäßen Verfahren ferner zu einer Umwandlung der Reaktionsmischung und dadurch bedingt zu einer Verringerung des epi-Feudomycins kommt. Massenspektrometrische Untersuchungen legen nahe, dass der Zucker abgespalten und der verbleibende Ring aromatisiert wird. Es wird weiterhin angenommen, dass es sich dabei um eine spezifische Zersetzung des epi-Feudomycins handelt, da Abbauprodukte von Epidaunorubicin nicht detektiert werden konnten. Insofern unterscheidet sich die im erfindungsgemäßen Verfahren erfolgende Zersetzung von der herkömmlichen sauren Hydrolyse der Anthrazycline, von der auch Epidaunorubicin betroffen wäre.

Das erfindungsgemäße Verfahren geht von Epidaunorubicin als Ausgangsmaterial aus, das in verschiedenen Schritten aufgereinigt wird. Dabei sind Herkunft und Herstellungsart des Epidaunorubicins nicht weiter eingeschränkt. So kann beispielsweise kommerziell erhältliches Epidaunorubicin eingesetzt werden, das einen Anteil an epi-Feudomycin enthält, der es für weitere Anwendungen ungeeignet macht.

In einer bevorzugten Ausführungsform des erfindungsgemäßen Aufreinigungsverfahrens wird das Epidaunorubicin der Mischung in Schritt a) mittels biotechnologischer Methoden, beispielsweise durch geeignete Mikroorganismen, gewonnen. Dabei liegt das Epidaunorubicin vorzugsweise zusammen mit epi-Feudomycin in der Fermentationsbrühe vor. Geeignete Mikroorganismen sind beispielsweise Bakterien aus der Gruppe der Actinobacteria, vor allem Stämme aus der Gruppe der *Streptomyces sp.,* beispielsweise *S. peucetius, S. coeruloruidus*, *S. griseus, Streptomyces sp. C5, S. peicetius var. caesius* und *S. bifurcus.* Ebenso können modifizierte Stämme oder Mutanten verwendet werden.

Vorzugsweise werden das Epidaunorubicin und das epi-Feudomycin der Mischung in Schritt a) des erfindungsgemäßen Verfahrens durch Extraktion aus der Fermentationsbrühe gewonnen. Die Extraktion kann dabei mehrere Schritte umfassen, beispielsweise die Extraktion mittels eines geeigneten Polymerharzes gefolgt von einer Flüssigextraktion. Die Mischung a) wird vorzugsweise aus dem eingeengten Konzentrat der Flüssigextraktion der Fermentationsbrühe und gegebenenfalls durch Zugabe des halogenhaltigen Lösungsmittels gewonnen.

In einer besonders bevorzugten Ausführungsform weist die Ausgangsmischung in Schritt a) einen basischen pH-Wert, besonders bevorzugt einen pH-Wert in einem Bereich von 8 bis 10,5 auf.

Das Epidaunorubicin liegt in der Mischung a) in gelöster Form in Gegenwart wenigstens eines halogenhaltigen Lösungsmittels vor. In einer bevorzugten Ausführungsform ist das halogenhaltige Lösungsmittel ausgewählt aus der Gruppe der chlorierten Lösungsmittel, insbesondere Chloroform (CHCl₃).

Der Gehalt an Alkoholen mit 1 bis 5 Kohlenstoffatomen in der Mischung der Schritte a) und b) des erfindungsgemäßen Verfahrens beträgt maximal 5 Vol.-%, bezogen auf das Gesamtvolumen der Mischung.

Es hat sich überraschend gezeigt, dass ein höherer Gehalt an Alkoholen mit 1 bis 5 Kohlenstoffatomen zu einer verringerten Reaktionsgeschwindigkeit führt, was sich wiederum negativ auf den Reinheitsgrad des Epidaunorubicins auswirkt.

Daher ist eine Ausführungsform der vorliegenden Erfindung bevorzugt, in der der Anteil an Alkoholen mit 1 bis 5 Kohlenstoffatomen in der Mischung der Schritte a) und b) maximal 4 Vol.-% beträgt, besonders bevorzugt 0,1 bis 4 Vol.-%, im Speziellen 1,0 bis 3 Vol.-%, jeweils bezogen auf das Gesamtvolumen der Mischung. Ein Alkoholgehalt im angegebenen, erfindungsgemäßen Bereich stellt sicher, dass das Epidaunorubicin vollständig in Lösung bleibt und die Reaktion in einem zufriedenstellenden Zeitrahmen abläuft.

Weiterhin bevorzugt ist eine Ausführungsform des erfindungsgemäßen Verfahrens, bei der der Alkohol mit 1 bis 5 Kohlenstoffatomen ausgewählt ist aus der Gruppe bestehend aus Methanol, Butanol, Propanol, Ethanol, Isopropanol, Pentanol, 2-Pentanol, 3-Pentanol, 2,2-Dimethylpropanol und Isobutanol. Im Speziellen ist eine Ausführungsform bevorzugt, in der der Alkohol mit 1 bis 5 Kohlenstoffatomen Methanol ist.

Weiterhin bevorzugt ist eine Ausführungsform, in der der Anteil an Wasser an der Mischung in Schritt a) maximal 1 Vol.-%, bezogen auf das Gesamtvolumen der Mischung, beträgt.

Es wurde weiterhin überraschend gefunden, dass das durch das erfindungsgemäße Verfahren gewonnene Epidaunorubicin einen besonders hohen Reinheitsgrad aufweist, wenn die Konzentration des Epidaunorubicin in der Mischung von Schritt a) nicht mehr als 13 g/L beträgt.

Daher ist eine Ausführungsform bevorzugt, bei der die Konzentration des Epidaunorubicins in der Mischung in Schritt a) maximal 13 g/L, bevorzugt 6 bis 13 g/L, insbesondere 8 bis 13 g/L beträgt. Es hat sich gezeigt, dass sich unerwünschte Nebenreaktionen und Störungen der Reaktion vermeiden lassen, wenn die Konzentration des Epidaunorubicins in der Mischung in Schritt a) in dem angegeben Bereich liegt. So konnte beispielsweise die Gefahr eines Ausfallens des Epidaunorubicins reduziert werden. Ein Ausfallen des Epidaunorubicins führt zu einem unerwünschten Ausbeuteverlust, da das ausgefallene Epidaunorubicin dem Reinigungsprozess entzogen wird. Darüber hinaus wurde beobachtet, dass zusammen mit dem Epidaunorubicin auch die abzutrennende Verunreinigung epi-Feudomycin ausfällt, so dass das Ausfällen keine geeignete Aufreinigungsmethode darstellt.

Gemäß Schritt b) des erfindungsgemäßen Verfahrens zur Aufreinigung von Epidaunorubicin wird der pH-Wert der Mischung auf einen Bereich von 5,0 bis 7,5 eingestellt. Es hat sich überraschend gezeigt, dass es zu einer Zersetzung des Epidaunorubicins kommt, wenn der pH-Wert oberhalb des erfindungsgemäßen Bereichs liegt. Wird der pH-Wert zu sauer, also auf einen Wert von unter 5 eingestellt, kommt es zu einer unerwünschten teilweisen Protonierung des Epidaunorubicins, was dazu führt, dass das Epidaunorubicin zusammen mit dem Feudomycin ausfällt und dem weiteren Prozessablauf entzogen wird.

Vorzugsweise erfolgt das Einstellen des pH-Werts der Mischung auf einen Bereich von 5,0 bis 7,5 mit Hilfe einer Säure, die über einen geeigneten pKa-Wert und gleichzeitig über eine gute Löslichkeit in dem halogenhaltigen Lösungsmittel, insbesondere Chloroform, verfügt. In einer bevorzugten Ausführungsform des erfindungsgemäßen Verfahrens erfolgt das Einstellen des pH-Wertes der Mischung in Schritt b) mit Hilfe einer oder mehrerer Säuren, vorzugsweise einer organischen Säure, besonders bevorzugt Essigsäure.

Weiterhin bevorzugt ist eine Ausführungsform des erfindungsgemäßen Verfahrens, in der die Menge der Säure 0,05 bis 0,3 Vol-%, vorzugsweise 0,1 bis 0,25 Vol.-%, bezogen auf das Gesamtvolumen der Mischung, beträgt. Nach Zugabe der Säure konnte eine deutliche Zunahme der Reaktionsgeschwindigkeit beobachtet werden. Liegt der Säuregehalt dagegen oberhalb von 0,3 Vol.-%, bezogen auf das Gesamtvolumen der Mischung, kann es zu Löslichkeitsproblemen kommen, die dazu führen, dass das Epidaunorubicin und das epi-Feudomycin aus der Lösung ausfallen und so dem weiteren Prozessvorgang entzogen werden.

In einer besonders bevorzugten Ausführungsform wird die Säure, die zum Einstellen des pH-Wertes der Mischung verwendet wird, vor der Zugabe in einem Alkohol mit 1 bis 5 Kohlenstoffatomen gelöst, vorzugsweise Methanol. Wie sich überraschend gezeigt hat, kann auf diese Weise Problemen betreffend die Löslichkeit des Epidaunorubicins vorgebeugt und ein Ausfallen des Epidaunorubicins verhindert werden. Dabei sollte der Gesamtgehalt an Alkohol mit 1 bis 5 Kohlenstoffatomen, insbesondere Methanol, in der Mischung den erfindungsgemäßen Wert von 5 Vol.-%, bezogen auf das Gesamtvolumen der Mischung, nicht übersteigen.

Gemäß Schritt c) des erfindungsgemäßen Verfahrens zur Aufreinigung von Epidaunorubicin wird die Mischung aus Schritt b), nach Einstellen des pH-Wertes auf den erfindungsgemäßen Bereich, auf über 50 °C erwärmt. Es wurde überraschend gefunden, dass sich die Aufreinigung beschleunigen lässt, wenn die Mischung auf eine Temperatur oberhalb von 25 °C erwärmt wird.

Vorzugsweise erfolgt eine Erwärmung der Mischung auf eine Temperatur, die dem Siedepunkt des halogenhaltigen Lösungsmittels entspricht. Als besonders geeignet haben sich dabei die erfindungsgemäßen Temperaturen von oberhalb von 50°C erwiesen, da ab dieser Temperatur eine deutliche Steigerung der Reaktionsgeschwindigkeit beobachtet werden konnte.

Daher ist eine Ausführungsform bevorzugt, in der die Mischung in Schritt c) auf eine Temperatur in einem Bereich von 55 bis 75 °C, vorzugsweise 60 bis 65 °C, erwärmt wird.

In einer bevorzugten Ausführungsform wird die Mischung in dem erfindungsgemäßen Verfahren für einen bestimmten Zeitraum bei einer Temperatur von über 25 °C, vorzugsweise bei einer Temperatur oberhalb von 35 °C, im Speziellen in einem Bereich von 60 bis 65 °C, gerührt. Dabei sollte die Zeitspanne in einem Rahmen liegen, der eine effiziente und zeitoptimierte Durchführung des erfindungsgemäßen Verfahrens erlaubt, wobei gleichzeitig ein befriedigender Reinheitsgrad des Epidaunorubicins erzielt wird.

Folglich ist eine Ausführungsform des erfindungsgemäßen Verfahrens bevorzugt, in der die Mischung in Schritt c) für einen Zeitraum von maximal 48 Stunden, vorzugsweise für einen Zeitraum von 10 bis 30 Stunden, besonders bevorzugt 15 bis 25 Stunden, gerührt wird. Eine Reaktionsdauer von mehr als 48 Stunden hat sich aus prozesstechnischer Sicht als nachteilig erwiesen, während bei einer Reaktionsdauer von weniger als 10 Stunden, die Abnahme des Anteils an epi-Feudomycin an der Mischung als nicht ausreichend beurteilt wurde. Besonders bevorzugt ist eine Ausführungsform, in der die Mischung in Schritt c) solange gerührt wird, bis der Gesamtgehalt an epi-Feudomycin weniger als 1 Gew.-%, bezogen auf das Gesamtgewicht des Epidaunorubicins, beträgt, sofern eine Reaktionsdauer von 48 Stunden nicht überschritten wird. Die Menge an epi-Feudomycin in der Mischung kann dabei beispielsweise mittels standardisierten Chromatographieverfahren, wie RP-18 HPLC, bestimmt werden.

Wie in Schritt d) des erfindungsgemäßen Verfahrens beschrieben, wird das Epidaunorubicin aufgereinigt. Diese Aufreinigung erfolgt vorzugsweise zu einem Zeitpunkt, an dem der Gesamtgehalt an epi-Feudomycin unterhalb eines Grenzwertes von 1 Gew.-%, bezogen auf das Gewicht des Epidaunorubicins, bestimmt mittels analytischer Chromatographie, abgesunken ist. Weiter bevorzugt erfolgt die Aufreinigung des Epidaunorubicins in Schritt d) mittels wässriger Extraktion.

Während der Aufreinigung des Epidaunorubicins in Schritt d) hat es sich als vorteilhaft erwiesen, wenn die wässrige Extraktion des Epidaunorubicins in Schritt d) im alkalischen durchgeführt wird. Daher ist eine Ausführungsform bevorzugt, in der die wässrige Extraktion des Epidaunorubicins in Schritt d) des erfindungsgemäßen Verfahrens bei einem pH-Wert von 8 bis 10, vorzugsweise 8,5 bis 9,5, erfolgt. Das Einstellen des pH-Wertes kann beispielsweise mit Hilfe von Ammoniak erfolgen, wobei es sich als besonders vorteilhaft erwiesen hat, wenn dieser etwa 0,5 - 1,5 Gew.% NaCl, so zum Beispiel etwa 1 Gew.-% NaCl, enthält, um einen teilweisen Übergang des Epidaunorubicins von der organischen Phase in die wässrige Phase zu verhindern. Die benötigte Menge an Ammoniak, um den gewünschten pH-Wert einzustellen, kann variieren und hängt von der zugegebenen Menge an Säure ab. So kann die Menge an Ammoniak, die benötigt wird, beispielsweise das 2,5fache der Menge an Säure in Gramm betragen.

Ein hoher Reinheitsgrad des Epidaunorubicins ist essentiell für eine weitere Umsetzung des Epidaunorubicins zu Epirubicin, da nur so dessen Ausbeute gesteigert werden kann. Daher ist eine Ausführungsform des erfindungsgemäßen Verfahrens bevorzugt, in dem sich Schritt d) ein weiterer Schritt e) anschließt, wobei es sich bei Schritt e) um eine chromatographische Reinigung des Epidaunorubicins handelt. Dabei erfolgt die chromatographische Reinigung vorzugsweise mit Kieselgel (SiO₂) als stationäre Phase, während als mobile Phase vorzugsweise ein Gemisch aus Methanol und Chloroform verwendet wird. Dies hat den Vorteil, dass kein Wechsel des Lösungsmittels vorgenommen werden muss, was wiederum zu der gesteigerten Effizienz des erfindungsgemäßen Verfahrens beiträgt.

Es wurde überraschend gefunden, dass die Beladung der Chromatographiesäule mit Epidaunorubicin, das nach dem erfindungsgemäßen Verfahren aufgereinigt wurde, im Vergleich zu Epidaunorubicin, das nach den herkömmlichen Verfahren gereinigt wurde, gesteigert werden konnte, ohne dass eine Verschlechterung der Trennleistung beobachtet wurde. Hier liegt ein weiterer Vorteil des erfindungsgemäßen Verfahrens, da eine höhere Beladung der Säule bei gleichbleibender Trennleistung einen effizienteren und ökonomischeren Prozessablauf ermöglicht. So hat sich überraschend gezeigt, dass die Beladung der Säule mit Epidaunorubicin, das gemäß dem erfindungsgemäßen Verfahren gereinigt wurde, auf bis zu 7 Gew.-%, bezogen auf das Trockengewicht der Säulenmatrix, gesteigert werden konnte, während bei herkömmlichen Verfahren die maximale Beladung der Säule bei etwa 4 Gew.-% liegt.

Weiterhin bevorzugt ist eine Ausführungsform des erfindungsgemäßen Verfahrens, bei der das Epidaunorubicin aus Schritt e) weiteren Reinigungsverfahren, beispielsweise einer Kristallisation unterzogen wird. Dabei kann die Kristallisation beispielsweise in Form des Hydrochloridsalzes erfolgen. Hierbei hat es sich gezeigt, dass die Qualität des gemäß dem erfindungsgemäßen Verfahren aufgereinigten Epidaunorubicins bereits nach einem Kristallisationsschritt so hoch war, dass auf den bei herkömmlichen Trennverfahren nach dem Stand der Technik üblichen zweiten Kristallisationsschritt verzichtet werden konnte.

Besonders bevorzugt ist eine Ausführungsform des erfindungsgemäßen Verfahrens, bei der das Verfahren die folgenden Schritte umfasst:
a) Bereitstellen einer Mischung enthaltend Epidaunorubicin, epi-Feudomycin und wenigstens ein halogenhaltiges Lösungsmittel, vorzugsweise Chloroform, wobei der Anteil an Feudomycin oberhalb von 1 Gew.-%, bezogen auf das Gesamtgewicht von Epidaunorubicin und epi-Feudomycin, liegt;
b) Einstellen des pH-Wertes der Mischung auf einen Bereich von 5,0 bis 7,5;
c) Erwärmen der Mischung aus Schritt b) auf eine Temperatur in einem Bereich von 55 bis 75 °C, vorzugsweise 60 bis 65 °C;
d) Aufreinigen des Epidaunorubicins, vorzugsweise mittels wässriger Extraktion;
e) Chromatographische Reinigung des Epidaunorubicins aus Schritt d),
wobei der Anteil an Alkoholen mit 1 bis 5 Kohlenstoffatomen an der Mischung der Schritte a) und b) 0,1 bis 4 Vol.-%, vorzugsweise 1,0 bis 3,0 Vol.-%, bezogen auf das Gesamtvolumen der Mischung, beträgt und der Anteil an Wasser an der Mischung in Schritt a) maximal 1 Vol.-%, bezogen auf das Gesamtvolumen der Mischung, beträgt.

Weiter bevorzugt ist eine Ausführungsform, in der das erfindungsgemäße Verfahren die folgenden Schritte umfasst:
a) Bereitstellen einer Mischung enthaltend Epidaunorubicin, epi-Feudomycin und wenigstens ein halogenhaltiges Lösungsmittel, vorzugsweise Chloroform, wobei der Anteil an epi-Feudomycin oberhalb von 1 Gew.-%, bezogen auf das Gesamtgewicht von Epidaunorubicin und epi-Feudomycin, liegt und die Konzentration des Epidaunorubicins in einem Bereich von 6 bis 13 g/L liegt;
b) Einstellen des pH-Wertes der Mischung auf einen Bereich von 5,0 bis 7,5;
c) Erwärmen der Mischung aus Schritt b) auf eine Temperatur in einem Bereich von 55 bis 75 °C, vorzugsweise 60 bis 65 °C;
d) Aufreinigen des Epidaunorubicins mittels wässriger Extraktion, wobei der pH-Wert der Extraktionsmischung in einem Bereich von 8 bis 10, vorzugsweise 8,5 bis 9,5, liegt;
e) Chromatographische Reinigung des Epidaunorubicins aus Schritt d),
wobei der Anteil an Alkoholen mit 1 bis 5 Kohlenstoffatomen an der Mischung der Schritte a) und b) 0,1 bis 4 Vol.-%, vorzugsweise 1,0 bis 3,0 Vol.-%, bezogen auf das Gesamtvolumen der Mischung, beträgt und der Anteil an Wasser an der Mischung in Schritt a) maximal 1 Vol.-%, bezogen auf das Gesamtvolumen der Mischung, beträgt.

In einer bevorzugten Ausführungsform sind die einzelnen Verfahrensschritt a) bis e) nicht austauschbar. Besonders bevorzugt erfolgt das Verfahren in der festgelegten Reihenfolge.

Die vorliegende Erfindung soll anhand der folgenden Beispiele näher erläutert werden, wobei diese keineswegs als Einschränkung des Erfindungsgedanken zu verstehen sind.

### Beispiele:

### Beispiel 1:

Die Konzentration an Epidaunorubicin in einer Mischung enthaltend Chloroform (CHCl₃), Epidaunorubicin und epi-Feudomycin wurde mittels Destillation auf einen Gehalt von 8 bis 12 g/L eingestellt. Eine entsprechende Ausgangsmischung kann beispielsweise durch Abtrennung der Chloroformphase aus der Fermentationsbrühe erhalten werden. Zu der aufkonzentrierten Lösung (128 L) wurden 194 g Essigsäure (0,15 Vol.-%) und 2,4 kg Methanol (2 Vol.-%) zugegeben, wobei sich die Angaben der Vol.-% jeweils auf das Gesamtvolumen der Lösung beziehen, und der pH-Wert so auf einen Bereich von 5,0 bis 7,5 eingestellt. Die erhaltene Lösung wurde auf 60 bis 65 °C erwärmt und für 17 Stunden in diesem Temperaturbereich gerührt. Dann wurde die Mischung auf 25 °C abgekühlt und der Gehalt der Verunreinigung epi-Feudomycin, bezogen auf das Gewicht von Epidaunorubicin mittels analytischer HPLC (RP18-HPLC) und Integration der gemessenen Peaks bestimmt. Das Ergebnis ist in Tabelle 1 wiedergegeben.

Beispiel 2 dient als Vergleichsbeispiel und zeigt das Ergebnis einer herkömmlichen Aufreinigung des Epidaunorubicins, bei der auf das Erwärmen der Mischung auf eine Temperatur von 60 bis 65 °C und die Zugabe von Essigsäure verzichtet wurde. Die Angaben des epi-Feudomycins beziehen sich jeweils auf die Menge an Epidaunorubicin.

**Tabelle 1:**

| Beispiel | epi-Feudomycin (%) |
|---|---|
| 1 | 0,6 |
| 2 (Vgl.) | 3,3 |

Wie Tabelle 1 zu entnehmen ist, konnte mittels des erfindungsgemäßen Verfahrens ein deutlich höherer Reinheitsgrad des Epidaunorubicin erreicht werden, der sich in einem geringeren Anteil an epi-Feudomycin wiederspiegelt.

In einem weiteren Schritt wurde das nach dem erfindungsgemäßen Verfahren aufgereinigte Epidaunorubicin mittels wässriger Extraktion bei einem pH-Wert von 8 bis 9 und anschließender chromatographischer Reinigung weiter behandelt. Dabei wurden verschiedene Beladungsmengen der Säule getestet. Die bei der chromatographischen Reinigung gewonnen Fraktionen wurden vereinigt und es wurde der Reinheitsgrad des Epidaunorubicins sowie der Anteile an epi-Feudomycin bestimmt. Die Ergebnisse sind in Tabelle 2 dargestellt. Dabei bezieht sich die Beladung der Säule auf das Gewichtsverhältnis von Epidaunorubicin zu dem Trockengewicht der Säulenmatrix, multipliziert mit 100%.

**Tabelle 2:**

| Beispiel | Beladung (%) | Reinheit (%) | Ausbeute (%) | Anteil epi-Feudomycin (%) |
|---|---|---|---|---|
| 3 | 5,1 | 91 | 87 | 0,3 |
| 4 | 5,8 | 86 | 97 | 0,2 |
| 5 | 7,2 | 90 | 97 | 0,2 |

Wie Tabelle 2 zu entnehmen ist, bleibt auch bei hoher Beladung der Säule die Trennleistung nicht nur unvermindert, sondern konnte sogar noch gesteigert werden.

Tabelle 3 zeigt die Ergebnisse von Versuchen, bei denen die Menge an Essigsäure, die zur Einstellung des pH-Wertes verwendet wurde, variiert wurde. Wie Tabelle 3 zu entnehmen ist, sinkt der Anteil an epi-Feudomycin bei zunehmender Menge an Säure. Als Lösungsmittel wurde Chloroform verwendet. Die Mischungen enthielten jeweils 1 Vol.-% Methanol und wurden jeweils bei 60 °C für 25 Stunden gerührt, bevor der Anteil an epi-Feudomycin bestimmt wurde.

Der Anteil an epi-Feudomycin an der Ausgangsmischung betrug 8,7%. Bei Beispiel 6 handelt es sich um ein Vergleichsbeispiel, bei dem der Mischung keine Essigsäure hinzugefügt wurde.

**Tabelle 3:**

| Beispiel | Essigsäure (Vol.-%) | epi-Feudomycin(%) |
|---|---|---|
| 6 | 0 | 7,7 |
| 7 | 0,1 | 3,3 |
| 8 | 0,2 | 1,9 |
| 9 | 0,3 | 1,3 |
| 10 | 0,5 | 0,8 |

Wie Tabelle 3 zeigt, führt bereits die Zugabe von 0,1 Vol.-% an Essigsäure zu einer Mischung enthaltend Chloroform, Epidaunorubicin, epi-Feudomycin und 1 Vol.-% Methanol zu einer deutlichen Abnahme der Verunreinigung epi-Feudomycin bei einer Reaktionsdauer von 25 Stunden bei einer Temperatur von 60 °C. Somit kann der Reinheitsgrad des erhaltenen Epidaunorubicins gesteigert werden, ohne dass es aufgrund aufwendiger Reinigungsmethoden zu Einbußen bei der Ausbeute kommt.

Wie den aufgeführten Beispielen zu entnehmen ist, führt das erfindungsgemäße Verfahren nicht nur zu einem deutlich höheren Reinheitsgrad des Epidaunorubicins im Vergleich zu herkömmlichen Reinigungsmethoden, sondern ermöglicht auch eine Steigerung der Effizienz und Ökonomie des Reinigungsprozesses durch eine höhere Säulenbeladung bei mindestens gleichbleibender Trennleistung sowie der Reduktion der erforderlichen Prozessschritte, wie beispielsweise durch die Entbehrlichkeit eines zweiten Kristallisationsschrittes.

## Patentansprüche

1. Verfahren zur Aufreinigung von Epidaunorubicin umfassend die folgenden Schritte:
a) Bereitstellen einer Mischung enthaltend Epidaunorubicin, epi-Feudomycin und wenigstens ein halogenhaltiges Lösungsmittel;
b) Einstellen des pH-Wertes der Mischung auf einen Bereich von 5,0 bis 7,5;
c) Erwärmen der Mischung aus Schritt b) auf über 50°C; und
d) Aufreinigen des Epidaunorubicins,
**dadurch gekennzeichnet,**
**dass** der Anteil an Alkoholen mit 1 bis 5 Kohlenstoffatomen an der Mischung in den Schritten a) und b) maximal 5 Vol.-%, bezogen auf das Gesamtvolumen der Mischung, beträgt.

2. Verfahren gemäß Anspruch 1,
**dadurch gekennzeichnet,**
**dass** der Anteil an Wasser an der Mischung in Schritt a) maximal 1 Vol-%, bezogen auf das Gesamtvolumen der Mischung, beträgt.

3. Verfahren gemäß Anspruch 1 oder 2, **dadurch gekennzeichnet,**
**dass** der Alkohol mit 1 bis 5 Kohlenstoffatomen ausgewählt ist aus der Gruppe bestehend aus Methanol, Butanol, Isopropanol, Ethanol, Propanol, Pentanol, 2-Pentanol, 3-Pentanol, 2,2-Dimethylpropanol und Isobutanol.

4. Verfahren gemäß einem oder mehrerer der Ansprüche 1 bis 3, **dadurch gekennzeichnet, dass** die Konzentration des Epidaunorubicins in Schritt a) 6 bis 13 g/L, vorzugsweise 8 bis 13 g/L beträgt.

5. Verfahren gemäß einem oder mehrerer der Ansprüche 1 bis 4, **dadurch gekennzeichnet, dass** das halogenhaltige Lösungsmittel ausgewählt ist aus der Gruppe der chlorierten Lösungsmittel, insbesondere Chloroform (CHCl₃).

6. Verfahren gemäß einem oder mehrerer der Ansprüche 1 bis 5, **dadurch gekennzeichnet, dass** das Einstellen des pH-Wertes in Schritt b) mit Hilfe einer oder mehrerer Säuren, vorzugsweise einer organischen Säure, besonders bevorzugt Essigsäure, erfolgt.

7. Verfahren gemäß Anspruch 6,
**dadurch gekennzeichnet,**
**dass** die Menge der Säure 0,1 bis 0,3 Vol-%, vorzugsweise 0,15 bis 0,25 Vol-%, bezogen auf das Gesamtvolumen der Mischung, beträgt.

8. Verfahren gemäß einem oder mehrerer der Ansprüche 1 bis 7,
**dadurch gekennzeichnet,**
**dass** die Mischung in Schritt c) auf eine Temperatur in einem Bereich von 55 bis 75°C, vorzugsweise 60 bis 65°C, erwärmt wird.

9. Verfahren gemäß einem oder mehrerer der Ansprüche 1 bis 8,
**dadurch gekennzeichnet,**
**dass** die Mischung in Schritt c) für einen Zeitraum von maximal 48 Stunden, vorzugsweise für einen Zeitraum von 10 bis 30 Stunden, besonders bevorzugt 15 bis 25 Stunden,
gerührt wird.

10. Verfahren gemäß einem oder mehrerer der Ansprüche 1 bis 9,
**dadurch gekennzeichnet,**
**dass** das Aufreinigen des Epidaunorubicins in Schritt d) mittels wässriger Extraktion erfolgt.

11. Verfahren gemäß Anspruch 10,
**dadurch gekennzeichnet,**
**dass** wässrige Extraktion des Epidaunorubicins in Schritt d) bei einem pH-Wert von 8 bis 10, vorzugsweise 8,5 bis 9,5, erfolgt.

12. Verfahren gemäß einem oder mehrerer der Ansprüche 1 bis 11,
**dadurch gekennzeichnet,**
**dass** sich Schritt d) ein weiterer Schritt e) anschließt, wobei es sich bei Schritt e) um eine chromtographische Reinigung des Epidaunorubicins handelt.

## Claims

1. A method for the purification of epidaunorubicin, comprising the following steps:
a) providing a mixture containing epidaunorubicin, epi-feudomycin and at least one halogen-containing solvent;
b) adjusting the pH value of the mixture to a range of from 5.0 to 7.5;
c) heating the mixture of step b) to above 50°C; and
d) purifying the epidaunorubicin,
**characterized in that**
the proportion of alcohols having 1 to 5 carbon atoms in the mixture in steps a) and b) is at most 5 vol.-%, based on the total volume of the mixture.

2. The method according to claim 1,
**characterized in that**
the proportion of water in the mixture in step a) is at most 1 vol.-%, based on the total volume of the mixture.

3. The method according to claim 1 or 2, **characterized in that**
the alcohol having 1 to 5 carbon atoms is selected from the group consisting of methanol, butanol, isopropanol, ethanol, propanol, pentanol, 2-pentanol, 3-pentanol, 2,2-dimethyl propanol, and isobutanol.

4. The method according to one or more of claims 1 to 3, **characterized in that**
the concentration of the epidaunorubicin in step a) is from 6 to 13 g/l, preferably from 8 to 13 g/l.

5. The method according to one or more of claims 1 to 4, **characterized in that**
the halogen-containing solvent is selected from the group of chlorinated solvents, in particular chloroform (CHCl₃).

6. The method according to one or more of claims 1 to 5, **characterized in that**
the adjustment of the pH value in step b) is performed by means of one or more acids, preferably an organic acid, particularly preferred acetic acid.

7. The method according to claim 6,
**characterized in that**
the amount of the acid is from 0.1 to 0.3 vol.-%, preferably from 0.15 to 0.25 vol.-%, based on the total volume of the mixture.

8. The method according to one or more of claims 1 to 7,
**characterized in that**
the mixture in step c) is heated to a temperature in a range of from 55 to 75°C, preferably from 60 to 65°C.

9. The method according to one or more of claims 1 to 8,
**characterized in that**
the mixture in step c) is stirred for a period of time of at most 48 hours, preferably for a period of time of from 10 to 30 hours, particularly preferred from 15 to 25 hours.

10. The method according to one or more of claims 1 to 9,
**characterized in that**
the purification of the epidaunoribicin in step d) is performed by means of aqueous extraction.

11. The method according to claim 10,
**characterized in that**
the aqueous extraction of the epidaunorubicin in step d) is performed at a pH value of from 8 to 10, preferably from 8.5 to 9.5.

12. The method according to one or more of claims 1 to 11,
**characterized in that**
step d) is followed by a further step e), step e) being a chromatographic purification of the epidaunorubicin.

## Revendications

1. Procédé de purification d'épidaunorubicine, comprenant les étapes suivantes :
a) préparation d'un mélange contenant de l'épidaunorubicine, de l'épi-feudomycine et au moins un solvant halogéné ;
b) ajustement du pH du mélange sur une plage de 5,0 à 7,5 ;
c) chauffage du mélange de l'étape b) à plus de 50 °C ; et
d) purification de l'épidaunorubicine,
**caractérisé en ce que**
la proportion d'alcools ayant de 1 à 5 atomes de carbone sur le mélange aux étapes a) et b) est au maximum de 5 % en volume, par rapport au volume total du mélange.

2. Procédé selon la revendication 1, **caractérisé en ce que** la proportion d'eau sur le mélange à l'étape a) est d'au maximum 1 % en volume, par rapport au volume total du mélange.

3. Procédé selon la revendication 1 ou 2, **caractérisé en ce que** l'alcool ayant de 1 à 5 atomes de carbone est choisi dans le groupe constitué du méthanol, du butanol, de l'isopropanol, de l'éthanol, du propanol, du pentanol, du 2-pentanol, du 3-pentanol, du 2,2-diméthylpropanol et de l'isobutanol.

4. Procédé selon l'une ou plusieurs des revendications 1 à 3, **caractérisé en ce que** la concentration de l'épidaunorubicine à l'étape a) est de 6 à 13 g/litre, de préférence 8 à 13 g/litre.

5. Procédé selon l'une ou plusieurs des revendications 1 à 4, **caractérisé en ce que** le solvant halogéné est choisi dans le groupe des solvants chlorés, en particulier le chloroforme (CHCl₃).

6. Procédé selon l'une ou plusieurs des revendications 1 à 5, **caractérisé en ce que** l'ajustement du pH à l'étape b) se fait à l'aide d'un ou de plusieurs acides, de préférence d'un acide organique, de manière particulièrement préférée de l'acide acétique.

7. Procédé selon la revendication 6, **caractérisé en ce que** la quantité d'acide est de 0,1 à 0,3 % en volume, de préférence de 0,15 à 0,25 % en volume, par rapport au volume total du mélange.

8. Procédé selon l'une ou plusieurs des revendications 1 à 7, **caractérisé en ce que** le mélange de l'étape c) est chauffé à une température dans une plage de 55 à 75 °C, de préférence 60 à 65 °C.

9. Procédé selon l'une ou plusieurs des revendications 1 à 8, **caractérisé en ce que** le mélange de l'étape c) est agité pendant une durée d'au maximum 48 heures, de préférence pendant une durée de 10 à 30 heures, de manière particulièrement préférée de 15 à 25 heures.

10. Procédé selon l'une ou plusieurs des revendications 1 à 9, **caractérisé en ce que** la purification de l'épidaunorubicine à l'étape d) s'effectue au moyen d'une extraction aqueuse.

11. Procédé selon la revendication 10, **caractérisé en ce que** l'extraction aqueuse de l'épidaunorubicine à l'étape d) s'effectue à un pH de 8 à 10, de préférence de 8,5 à 9,5.

12. Procédé selon l'une ou plusieurs des revendications 1 à 11, **caractérisé en ce qu'**une autre étape e) s'adjoint à l'étape d), l'étape e) étant une purification chromatographique de l'épidaunorubicine.
